(19) Europäisches Patentamt
European Patent Office
Office européen des brevets

(11) Veröffentlichungsnummer : **0 023 592**
**B1**

# (12) EUROPÄISCHE PATENTSCHRIFT

(45) Veröffentlichungstag der Patentschrift : **30.03.83**

(51) Int. Cl.[3] : **C 07 D209/88**

(21) Anmeldenummer : **80103887.8**

(22) Anmeldetag : **08.07.80**

## (54) Verfahren zur Herstellung von 4-Hydroxy-carbazol.

(30) Priorität : **14.07.79 DE 2928483**

(43) Veröffentlichungstag der Anmeldung : **11.02.81 Patentblatt 81/06**

(45) Bekanntmachung des Hinweises auf die Patenterteilung : **30.03.83 Patentblatt 83/13**

(84) Benannte Vertragsstaaten : **AT BE CH DE FR GB IT LI LU NL SE**

(56) Entgegenhaltungen :
**DE C 2 240 599**
**BEILSTEINS HANDBUCH DER ORGANISCHEN CHEMIE, 4. Auflage, 3. und 4. Ergänzungswerk, Band 20, Teil 6, 1978, SPRINGER-VERLAG, Berlin, Heidelberg, New York Seiten 3824 bis 3825**
**Chemical Abstracts Band 50, Nr. 10 25. Mai 1956, Columbus, Ohio, USA J.A. CUMMINS et al. « Hydroxycarbazoles and tetrahydrohydroxycarbazoles III. » Spalte 7102 g**
**Synthetic Reagents, Volume II, s.s. Pizey, Seite 207**

(73) Patentinhaber : **BOEHRINGER MANNHEIM GMBH**
**Sandhofer Strasse 112-132 Postfach 31 01 20**
**D-6800 Mannheim 31-Waldhof (DE)**

(72) Erfinder : **Lauer, Karl, Dr. rer. nat.**
**Strahlenburg**
**D-6905 Schriesheim (DE)**
Erfinder : **Kiegel, Einhart, Dr. rer. nat.**
**Gluckstrasse 4**
**D-6800 Mannheim-1 (DE)**

**EP 0 023 592 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Verfahren zur Herstellung von 4-Hydroxy-carbazol

Die vorliegende Erfindung betrifft ein verbessertes Verfahren zur Herstellung von 4-Hydroxy-carbazol, welches ein wichtiges Ausgangsprodukt für die Gewinnung pharmakologisch wirksamer Carbazol-Derivate ist (vgl. DBP 22 40 599).

Es bieten sich verschiedene Möglichkeiten zur Synthese von 4-Hydroxy-carbazol an :

1. Aus Bromnitrobenzol und Jodanisol.

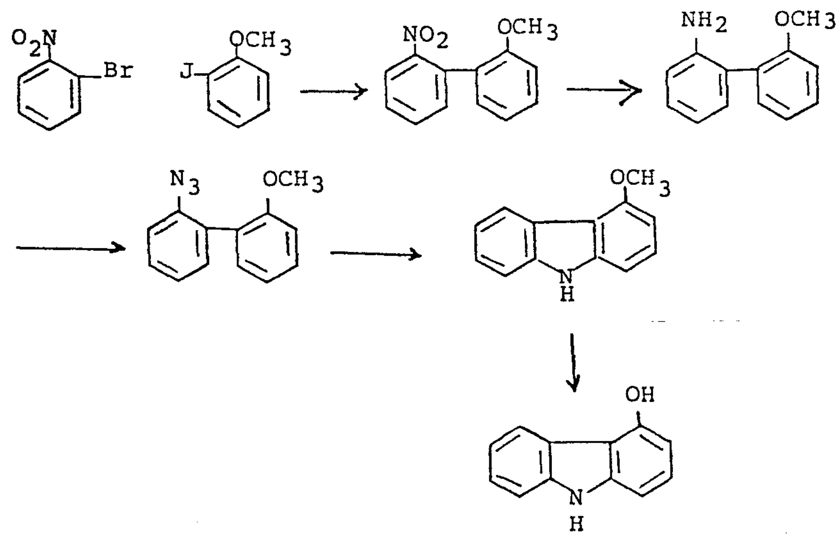

Dieses Verfahren ist jedoch für eine wirtschaftliche Herstellung von 4-Hydroxy-carbazol wenig geeignet, weil die Ausgangsmaterialien sehr teuer sind und die Durchführung des Verfahrens auf erhebliche verfahrenstechnische Schwierigkeiten stößt.

2. Aus Chlornitrobenzol via Dinitrobiphenyl.

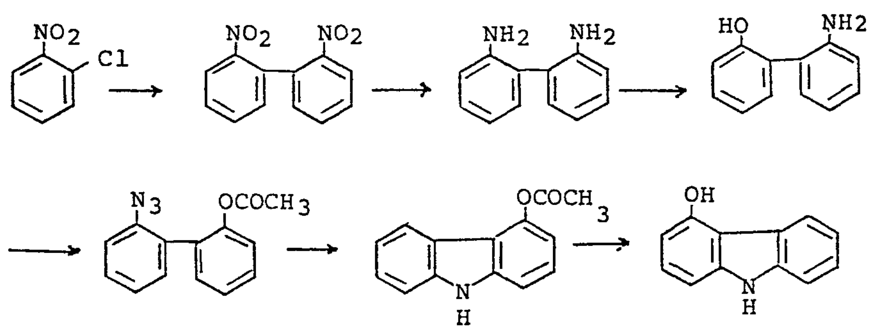

Diese 6-Stufen-Synthese hat ebenfalls erhebliche Nachteile. Infolge der Gefährlichkeit einer Zwischenstufe, bei der mit Stickstoffwasserstoffsäure gearbeitet wird, sind besondere Vorsichtsmaßnahmen erforderlich. Auch ist der übrige Aufwand groß, so daß die Kosten beträchtlich sind.

Zur Lösung der Aufgabenstellung — Produktion größerer Mengen 4-Hydroxy-carbazol, war es somit notwendig, ein Verfahren zu entwickeln, das wirtschaftlich und in technischem Maßstab durchführbar ist. Dieses Verfahren wird im folgenden näher beschrieben :

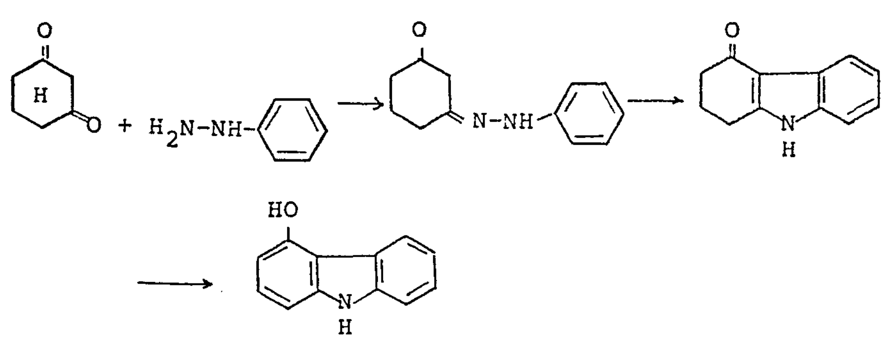

Cyclohexandion wird mit Phenylhydrazin zum Mono-phenylhydrazon umgesetzt und dieses im Sinne einer Fischer-Umlagerung in das 1,2,3,4-Tetrahydro-4-oxocarbazol überführt (Ann. *278*/1894, S. 39 ; Soc. *1951*, S. 700). Anschließend erfolgt eine Dehydrierung zum 4-Hydroxy-carbazol. Ganz entscheidend für eine wirtschaftliche Durchführung dieses Verfahrens ist die Auffindung einer geeigneten Dehydrierungsmethode bzw. eines geeigneten Dehydrierungskatalysators.

Mehrere literaturbekannte Dehydrierungs- bzw. Aromatisierungsmethoden wurden getestet, wie z. B.

a) Dehydrierung durch Bromieren/Dehydrobromieren
b) Dehydrierung mit Schwefel
c) Dehydrierung mit $LiCl/CuCl_2$ (TH-Letters *1977*, 821)
d) Dehydrierung mit Braunstein
e) Dehydrierung mit $PbO_2$
f) Dehydrierung mit Chloranil

Alle diese Methoden verliefen unbefriedigend.

Ferner ist die Dehydrierung von Tetrahydro-oxoindol mit Pd/C in J. heterocyclische Chemie *14*, 71 (1977) und Chim. Ther. *1970*, 279 beschrieben, wobei jedoch erhebliche Mengen — bis zu 50 Gewichtsprozent — des teuren Pd/C-Katalysators, bezogen auf das Ausgangsmaterial, notwendig sind und die Reaktion nur in hochsiedenden Lösungsmitteln — beispielsweise Cymol — gut abläuft.

Dehydrierungen bzw. Aromatisierungen mit Raney-Nickel als Katalysator sind ebenfalls bekannt. So wird z. B. aus 1,2,3,4-Tetrahydrocarbazol durch Raney-Nickel in Xylol unter Kochen am Rückfluß Carbazol hergestellt (Badcock et al., Soc. *1951*, 1373). Reduktionen von Ketonen zu Alkoholen in wässrigalkalischer Lösung mit Raney-Nickel sind in « Synthetic Reagents » Volume II beschrieben, jedoch entstehen bei erhöhten Temperaturen durchweg die unerwünschten Kohlenwasserstoffe.

Es wurde nun überraschend gefunden, daß die Dehydrierung von Tetrahydrooxocarbazol schon bei 100 °C in wäßriger, alkalischer Lösung mit Raney-Nickel als Katalysator abläuft. Dies war nicht zu erwarten, weil sich das Ausgangsmaterial in wäßrigem Alkali kaum löst. Ferner überrascht die geringe Bildung von Carbazol.

Gegenstand der vorliegenden Erfindung ist ein Verfahren zur Herstellung von 4-Hydroxycarbazol durch Dehydrierung von 1,2,3,4-Tetrahydro-4-oxo-carbazol, dadurch gekennzeichnet, daß man in wäßrigalkalischer Lösung mit Raney-Nickel als Katalysator arbeitet.

Zur Durchführung des Verfahrens wird das Tetrahydrooxocarbazol in wäßriger Alkalilauge aufgenommen, insbesondere Kalilauge oder Natronlauge. Die Alkalilauge wird in der 4-8fachen, vorzugsweise 5-6fachen stöchiometrischen Menge, bezogen auf die eingesetzte Menge 1,2,3,4-Tetrahydro-4-oxo-carbazol, verwendet. Vorteilhaft wird mit ungefähr 2 n Alkalilauge gearbeitet. Zweckmäßig arbeitet man in einer Inertgas-Atmosphäre, z. B. unter Stickstoff. Die Reaktion erfolgt unter Rückflußkochen und dauert 50-70 Stunden. Zur Isolierung des Produkts wird die alkalische Reaktionslösung vom Katalysator abgetrennt und angesäuert, wobei das Produkt in schleuderfähiger Form ausfällt.

Besonders günstig ist die wirtschaftliche Seite des neuen Verfahrens, da die Herstellkosten nur etwa 1/10 derjenigen der oben unter 2) geschilderten Methode betragen.

Im nachstehenden Beispiel ist das erfindungsgemäße Verfahren näher erläutert.

## Beispiel

Chemikalien :

60 kg Kaliumhydroxid (KOH)
30 kg 1,2,3,4-Tetrahydro-4-oxo-carbazol (THOC)
21 kg Raney-Nickel-Katalysator, feucht (Typ B 113 B) halbkonzentrierte Salzsäure.

In einen sauberen 1.200 l-$V_4A$-Apparat werden nacheinander

450 l Wasser,
60 kg KOH-Plätzchen (p.a. Ware) und
30 kg THOC eingefüllt.

(Vor der Zugabe von THOC sollen die KOH-Plätzchen gelöst sein).

Dann wird die Apparatur verschlossen, zweimal evakuiert und mit Stickstoff wieder belüftet.

Der Katalysator wird hinzugegeben und anschließend noch einmal mit Stickstoff gespült. Unter Rühren wird schwach am Rückfluß gekocht. Die Reaktionszeit beträgt ca. 60-64 Stunden.

Nach dem Reaktionsende wird auf Raumtemperatur abgekühlt und die Reaktionslösung abfiltriert. Unter Rühren wird mit halbkonzentrierter Salzsäure die stark alkalische Lösung auf pH 12,1 eingestellt (pH-Meter). Das ausgefallene THOC (ca. 2 kg) wird abgesaugt.

Die abgesaugte Lösung wird wiederum in den 1.200 l-$V_4A$-Apparat gegeben und dort unter Rühren durch Zugabe von halbkonzentrierter Salzsäure auf pH 1 gestellt (die Zugabe der Salzsäure soll langsam

erfolgen). Das ausgefallene Produkt wird abgeschleudert und mit Wasser säurefrei gewaschen. Anschließend wird bei 60 °C im Vakuumtrockenschrank getrocknet. Man erhält ca. 24-25 kg 4-Hydroxycarbazol vom Schmelzpunkt 163-164 °C.

Zur Herstellung des als Ausgangsprodukts verwendeten 1,2,3,4-Tetrahydro-4-oxo-carbazols (THOC) geht man wie folgt vor :

1. Cyclohexandion-1,3-monophenylhydrazon

Chemikalien :

40,0 kg Cyclohexandion-1,3
38,7 kg Phenylhydrazin
550 l Äthanol
3,4 kg A-Kohle (Brilonit Fx pur)

40 kg Cyclohexandion-1,3 werden bei 20 °C in einem 1.200 l-Emaille-Apparat in 250 l Wasser gelöst. Danach wird zweimal evakuiert und mit Stickstoff entspannt. Über das Zulaufgefäß läßt man bei einer Innentemperatur von 20-25 °C innerhalb von 4 Stunden eine Lösung von 38,7 kg Phenylhydrazin in 500 l Wasser hinzulaufen. Dabei läßt man zunächst in 20 Minuten ca. 50 l hinzulaufen und rührt 30 Minuten. Man überprüft mittels einer Probe die Kristallisation. Dann wird die Gesamtmenge innerhalb von 4 Stunden hinzugegeben. Anschließend wird der Ansatz 3 Stunden nachgerührt. Das sandig anfallende Phenylhydrazon wird geschleudert und mit 350 l Wasser gewaschen.

Das Rohprodukt wird im Umlufttrockenschrank bei 60 °C getrocknet. Ausbeute : 70 kg. Das getrocknete Rohprodukt wird aus der 6,7 fachen Menge (ca. 500 l) Äthanol unter Zusatz von 3,4 kg Aktivkohle umkristallisiert. Nach der Trocknung bei 60 °C erhält man 46 kg des gewünschten Phenylhydrazons vom Schmelzpunkt 187-189 °C.

2. Cyclisierung

Chemikalien :

22 kg Cyclohexandion-1,3-monophenylhydrazon
110 kg Zinkchlorid
130 l Eisessig
Äthanol
A-Kohle (Brilonit Fx pur)

In einen trockenen, sauberen 500 l-Emaille-Apparat werden 130 l Eisessig und 110 kg Zinkchlorid eingetragen. Danach werden 20 l Eisessig bei Normaldruck abdestilliert. Bei einer Innentemperatur von 60-70 °C werden 22 kg des Phenylhydrazons eingetragen. Man heizt mit Normaldruck-Dampf auf 90-100 °C an und hält dann die Temperatur mit einem Warmwasserkreislauf. Die Reaktion ist exotherm (sollte die Innentemperatur über 110 °C steigen, wird kurz gekühlt). Man erhält den Apparateinhalt insgesamt ca. 4 Stunden bei 90-100 °C. Nach 4 Stunden wird auf 75-80 °C abgekühlt und die heiße Lösung in insgesamt 1 100 l Wasser eingerührt. Man rührt eine Stunde nach. Das Rohprodukt wird abgeschleudert und mit ca. 500 l Wasser weitgehend chloridfrei gewaschen. Nach dem Trocknen im Umlufttrockenschrank bei 60 °C erhält man ca. 17 kg 1,2,3,4-Tetrahydro-4-oxo-carbazol.

Die Umkristallisation erfolgt aus Äthanol (ca. 1 : 13), (klare, dunkle Lösung) unter Zusatz von 10 % Brilonit Fx pur.

Ausbeute : 12-13 kg ; Schmelzpunkt : 220-221 °C.

**Ansprüche**

1. Verfahren zur Herstellung von 4-Hydroxycarbazol durch Dehydrierung von 1,2,3,4-Tetrahydro-4-oxo-carbazol, dadurch gekennzeichnet, daß man mit Raney-Nickel als Katalysator in wässriger Alkalilauge arbeitet, die in der vier- bis achtfachen stöchiometrischen Menge, bezogen auf das eingesetzte 1,2,3,4-Tetrahydro-4-oxocarbazol, verwendet wird.

2. Verfahren gemäß Anspruch 1, dadurch gekennzeichnet, daß die Alkalilauge in der fünf- bis sechsfachen stöchiometrischen Menge, bezogen auf das eingesetzte 1,2,3,4-Tetrahydro-4-oxo-carbazol, verwendet wird.

3. Verfahren gemäß Anspruch 1 und 2, dadurch gekennzeichnet, daß man als Alkalilauge Kalilauge verwendet.

4. Verfahren gemäß Ansprüchen 1 bis 3, dadurch gekennzeichnet, daß man in einer Inertgas-Atmosphäre arbeitet.

## Claims

1. Process for the preparation of 4-hydroxy-carbazole by the dehydrogenation of 1,2,3,4-tetrahydro-4-oxo-carbazole, characterised in that one operates with Raney nickel as catalyst in aqueous alkali metal hydroxide solution, which is used in a four to eightfold stoichiometric amount, referred to the 1,2,3,4-tetrahydro-4-oxo-carbazole employed.

2. Process according to claim 1, characterised in that the alkali metal hydroxide solution is employed in a five to sixfold stoichiometric amount, referred to the 1,2,3,4-tetrahydro-4-oxo-carbazole employed.

3. Process according to claims 1 and 2, characterised in that one uses potassium hydroxide solution as alkali metal hydroxide solution.

4. Process according to claims 1 to 3, characterised in that one operates in an inert gas atmosphere.

## Revendications

1. Procédé de fabrication de 4-hydroxycarbazole par déshydrogénation de 1,2,3,4-tétrahydro 4-oxo-carbazole, caractérisé en ce que l'on conduit la réaction en solution alcaline aqueuse avec du nickel de Raney comme catalyseur, la quantité de solution alcaline étant comprise entre 4 et 8 fois la quantité stœchiométrique calculée par rapport au 1,2,3,4-tétrahydro 4-oxo-carbazole mis en œuvre.

2. Procédé selon la revendication 1 caractérisé en ce que la quantité de solution alcaline mise en œuvre est comprise entre 5 et 6 fois la quantité stœchiométrique, calculée par rapport au 1,2,3,4-tétrahydro 4-oxo-carbazole.

3. Procédé selon les revendications 1 et 2 caractérisé en ce que la solution alcaline est une solution d'hydroxyde de potassium.

4. Procédé selon les revendications 1 à 3 caractérisé en ce que l'on travaille sous une atmosphère de gaz inerte.